# EUROPEAN PATENT SPECIFICATION

(11) **EP 1 502 587 B1**
(45) Date of publication and mention of the grant of the patent: **23.08.2006**
(21) Application number: 03386019.8
(22) Date of filing: 30.07.2003
(51) Int. Cl.: A61K 9/48, A61K 31/137

(54) **Sustained release formulation for Venlafaxine hydrochloride**
Venlafaxine Hydrochloridformulierungen mit verzögerter Freisetzung
Formulation à libération prolongée du chlorhydrate de Venlafaxine

(43) Date of publication of application: 02.02.2005
(73) Proprietor: Pharmathen S.A., Pallini, Attikis 15351 (GR)
(72) Inventor: Karavas, Evangelos, Agios Dimitrios Attikis 173 42 (GR); Lioumis, Konstantinos, Keratea Attikis 19001 (GR); Politis, Stavros, Athens 11527 (GR)

(56) References cited:
- WO-A1-03/013480
- WO-A2-99/22724

## Description

The rapid initial release of the water-soluble drug substances from matrix delivery systems is a well-known phenomenon. Venlafaxine HCL is a drug substance that is very soluble in water (more than 1000mg are dissolved in 1ml of water), so the said phenomenon is observed when the release of this drug substance from simple matrix systems is studied.

Another parameter very important for the pharmaceutical industry is the achievement of a linearity between the strength and the formulation. It is well known that drug delivery from matrix systems that are tablets is highly affected by the geometrical characteristics of the tablet-matrix. This phenomenon prevents the achievement of linearity between the different strengths of a drug product and the total weight of the said dosage form. As the strength gets bigger and the size of the matrix increases the dissolution rate is delayed, so the dosage forms that refer to different strengths of the same drug product do not exhibit the same dissolution profile. Linearity between the strength and the formulation of a dosage form without the release characteristics being influenced is highly desired in the pharmaceutical industry for manufacturing, pharmacokinetic and economical reasons.

An objective of the present invention is to provide a sustained release formulation which is free of the increased release of the drug observed at the initial stages of release that occurs in sustained release systems containing water soluble drugs such as venlafaxine HCl, known as burst phenomenon.

Another objective of the present invention is to provide a sustained release formulation capable of delivering the drug substance within 24 hours and is therefore suitable for once daily administration of the said drug substance.

Another objective of the present invention is to provide a sustained release formulation that exhibits linearity between the strength of the drug formulation and the total mass of the formulation, by proportional increase of the amounts of the drug substance and the excipients in the formulation.

The dosage form described in the present invention may be divided into smaller doses. This is desired in antidepressant medication treatments, where the therapy is tailored for each individual patient requirement.

Little formulation work has been conducted to date in order to overcome both this release problem concerning water-soluble dug substances such as Venlafaxine HCl and at the same time achieve linearity between strength and formulation as described above. Zero order kinetics are considered an optimal rate for drug delivery from sustained release systems. It is very usual though a rapid release of the drug to be observed during the first hours of release. This rapid initial drug release results to significant deviation from the desired zero order kinetics. This deviation affects the drug plasma concentrations resulting to a higher risk of occurrence of side effects, while effectiveness is deteriorated.

EP700289 describes a type of tablet known as osmotic pump.

EP1253910 also describes an osmotic pump.

EP 178780 describes a multiparticulate controlled release selective serotonin reuptake inhibitor (SSRI) formulation for oral administration, which comprises pellets coated with rate-controlling polymer, which allows controlled release of the SSRI over a period of not less than 12h.

WO0224160 describes a formulation of Long Acting Antidepressant Microparticles.

EP1028718 and EP0797991 describe encapsulated formulations of spheroid particles as sustained release formulation containing Venlafaxine.

EP1157690 describes a sustained release pharmaceutical composition free of food effect. The composition claimed in this patent is a single double-coated tablet made of compressed granules. Venlafaxine is mentioned as an example of drug substance the absorption of which is known to be influenced by food intake. S.Troy et al., Current Therapeutic Research, VOL.58, NO 8, pp 504-514 performed pharmacokinetic studies in order to assess the effect of food intake on the pharmacokinetic disposition venlafaxine and its active metabolite O-desmethoxyvenlafaxine (ODV). In two studies, Venlafaxine sustained release 75 and 150 mg formulations were administered to healthy subjects in a fasted state or a high fat meal. The studies were conducted with a two period cross over study design. The administration of Venlafaxine sustained release 75 or 150 mg capsules with a fat meal did not affect the rate or extent of Venlafaxine absorption compared with administration to the fasting condition.

In all the above-mentioned patents the osmotic pump type or formulations made from microparticles or spheroids are suggested. However, these types of formulations require both higher cost of production and more sophisticated equipment in relation to more conventional types (e.g. tablets) and at the same time more complicated and thus more time consuming production process. On the other hand, the desired zero order release kinetics is not always achieved.

WO9847491 describes an extended release dosage composition in the form of a tablet matrix, comprising of a drug substance and a combination of a hydrophilic and hydrophobic polymer of well-known groups used for controlled drug delivery formulations. In this document different ratios of the hydrophilic-hydrophobic polymer, as well as channeling agents and surfactants are used in order to modify the wettability of the described matrix, in order to combine it with a drug substance of a given solubility in aqueous systems. This patent is not specifically developed for Venlafaxine HCl and the described dosage form cannot provide linearity between the strength and the formulation of the dosage form, without affecting the release characteristics of the drug substance.

None of the above documents teaches or suggests the present invention.

### SUMMARY OF THE INVENTION:

The present invention provides a process for reducing the initial rapid release of the water-soluble drug substance Venlafaxine HCl from a formulation according to the present invention, using one or more functional cores coated with a functional coating layer or film that limits the surface of the core(s) that is available for drug release during the initial stages of the drug delivery.

Figure 1 shows a description of the application of the functional coating layer.

Figure 2 shows the comparative dissolution of the cores from examples 2.1 to 2.6.

### Detailed description:

- The present invention consists in a multi tablet capsule delivery system.

- Each capsule of said delivery system contains 1-6 mini tablets, containing a pharmacologically active water-soluble substance, Venlafaxine HCl.

- Each one of said tablets comprises of a functional core, which is partially or totally coated with an appropriate coating agent, so that the surface of the core that is initially available for drug delivery is limited.

- The core comprises
i. The drug substance, Venlafaxine HCl, in a proportion that varies between 10-40%by weight
ii. 40-80% of a gelling agent. This gelling agent can be chosen among; hydroxypropylmethylcellulose, hydroxypropylcellulose, hydroxycellulose phthalate, poly(ethyleneoxide), polylactic acid, xanthan gum, alginates, sodium and calcium carboxymethylcellulose, carragheen, carbomer, carbopol (oral use), methylhydroxyethylcellulose, propylhydroxyethylcellulose, polyhema, methylcellulose, alginates and other swellable poymers. The swelling agent used in the formulation should preferably be of high viscosity, as the incorporated drug substance is highly soluble in water and the diffusion rate through the gelling agent should be limited, without beholding the drug substance after the desired time window.
iii. 30-60% of a non-swellable (also characterized as monolithic or plastic) agent or system comprising of one or a mixture of water insoluble, non-swelling polymers such as: ethyl cellulose, cellulose acetate propionate, cellulose acetate, poly(ethyl acrylate, methyl methacrylate, trimethylammonioethyl methacrylate chloride) 1:2:0.1, commerced as Eudragit RS 100® poly(ethyl acrylate, methyl methacrylate, trimethylammonioethyl methacrylate chloride) 1:2:0.2 copolymer, commercially available as Eudragit RL®, polyvinylpyrrolidone acetate, polyvinyl chloride, polyvinyl acetate, polyethylene, and others. The function of these compounds is to limit the swelling rate of the gelling agent and to reduce the penetration of water through the pores that are formed by the swelling of the gelling agent and the diffusion of the drug substance from the core.
iv. A conjugation agent, a surfactant or a polymer that forms bonds between the gelling agent and the non swellable agent, or between the drug substance and the gelling or the non swellable agent, causing interactions between the constituents of the core that limit its swelling properties. Surfactants that are used as conjugation agents are usually anionic, as sodium lauryl sulphate, sodium docusate, sodium cetostearyl sulphate and triethanolamine lauryl sulphate, in proportions 2-12% by weight. Non ionic compounds, such as polysorbates exhibit weak conjunction ability, while cationic surfactants do not have such properties. Polymers used as binding agents between the gelling agent and the drug substance are polyvinylpyrrolidone, polyvinyl alcohol and polyvinylpyrroliudone acetate, in proportions of 10-30% by weight.
v. 1-30% by weight of classical excipients such as:
   a. Lubricants and glidants, as Mg, Ca and Zn Stearate, silicon dioxide, talc and stearic acid, or any other insoluble in water lubricant or glidant.
   b. Binders: the binders adopted for the invention should not be feely soluble in water. For example, polyvinylpyrrolidone acetate is preferred over polyvinylpyrrolidone as it obtains sustained release properties and enhances the relevant behavior of the core.
   c Diluents: any diluents free of disintegrating properties such as talc, dicalcium phosphate and calcium sulphate dihydrate could be adopted.

The classical excipients used for the preparation of the core should exhibit low solubility in water and free of disintegrant properties.

The core can be obtained by either a direct compression process, or through a wet granulation and compression process.

- In order to optimize the cohesiveness of the core a wet granulation process step is essential. The gelling agent(s), the active ingredient, the non-swelling polymers and the conjugation agent(s) are mixed together, comprising the internal phase to be submitted to the wet granulation step. The solvent used for the wet granulation step could be any suitable solvent for use in the manufacture of oral dosage forms. The solvent or mixture of solvents should be able to dilute or disperse the drug substance, the swellable polymers, the non-swellable polymers and the conjunction agent, so that the interactions between the above compounds can be developed. Such solvents are ethanol, acetone, isopropyl alcohol, water and mixtures of the said solvents. Alternatively the non-swelling compound is dissolved into an appropriate co-solvent preparing a 5-40% solution or uniform dispersion that is used for the wet granulation step of the rest of the constituents of the internal phase. The conjunction agent may also be diluted or dispersed in the granulation fluid. Any diluents or binders may be added in the internal phase.

- After drying, the granule mass is mixed with the excipients comprising the external phase (glidants, lubricants and binders) and the granular/powder mixture is compressed into tablets.

- The core is partially or totally coated by a coating layer or a coating film that reduces the initial rapid release of the water-soluble drug substance from the core, via two mechanisms.
i. By reducing the surface of the core that is initially available for the release of the drug substance, during the initial stages of the wetting of the dosage form of the formulation.
ii. By suppressing the core and in particular the swellable gelling agents. This way the penetration of water through the core that causes the diffusion of the drug substance and its rapid release during the initial steps of the wetting of the core is limited and the "burst" phenomenon is restricted.

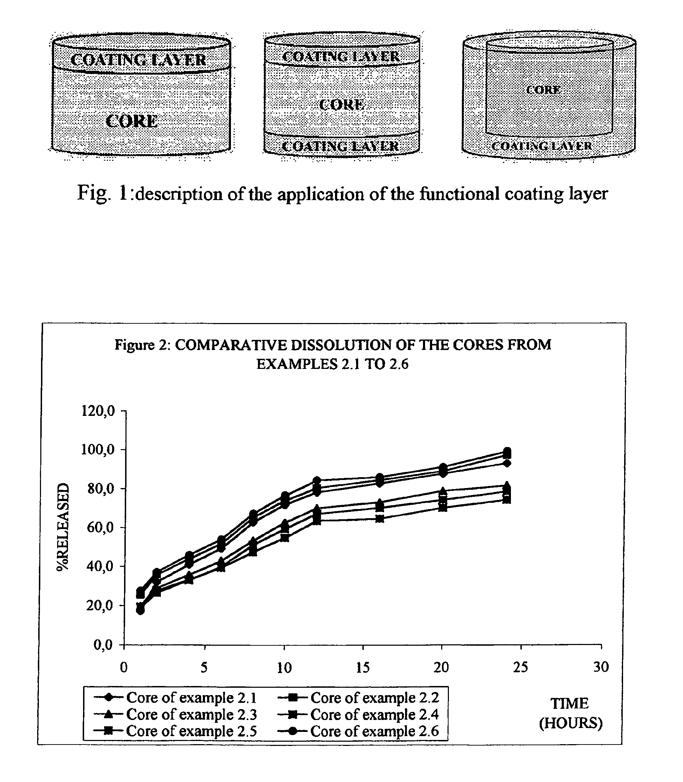

- The coating layer is applied on (as described in figure 1):
i. One surface of the core with a thickness that ranges between 3-30% of the diameter of the core, providing a two-layer tablet.
ii. Two surfaces of the core with a thickness that ranges between 3-30% of the diameter of the core, providing a three-layer tablet.
iii. One surface and the perimeter of the core with a thickness that ranges between 3-30% of the diameter of the core, forming a "cap" that covers the larger part of the core, leaving only one flat surface for the release of the drug substance.

- The coating layer comprises a polymer and a water-soluble compound. The polymer can be a swelling agent or a non-swelling agent, similar to the ones used for the core. The water soluble compound can be:
i. A water soluble salt such as sodium chloride, sodium bicarbonate, or any other water soluble salt that can be used as an excipient in a solid oral pharmaceutical formulation.
ii. A water soluble small organic compound like mannitol, lactose, sucrose, sorbitol, citric acid or any other water soluble, low relative molecular mass organic compound that can be used as an excipient in a solid oral pharmaceutical formulation.
iii. A water-soluble polymer like polyvinylpyrrolidone, polyvinyl alcohol, low viscosity hydroxyprolylmethyl cellulose, or any other water-soluble polymer that can be used as an excipient in a solid oral pharmaceutical formulation.

During the initial stages of the wetting of the coating layer the water-soluble compounds dissolve rapidly, creating pores through the drug substance can be diffused and released.

The polymer reduces the diffusion of the drug substance by reducing the surface of the core that is available for the dissolution of the drug substance.

The function of the coating layer is time limited with an optimal duration from 0 up to 2-4 hours of the drug release.

The function of the coating layer is advanced and terminated through two different mechanisms, depending on the kind of polymer that is enabled:
i. In the case of swellable polymers the wetting of the polymer causes the formation of moving boundaries delimiting different physical conditions inside the matrix of the coating layer (dry coating material, swollen polymer, dissolved/undissolved polymer). The polymer swells through a swelling front that is followed by a diffusion front, through which soluble compounds can be diffused through the mass of the polymer and be released and an eroding front through which the polymer dissolves into the surrounding fluids. The termination of the function of the coating layer consisting of swellable polymers coincides with the extension of the diffusion layer up to the surface of the core. After that stage the surface of the core that was covered by the polymer layer can be hydrated and the drug substance can be diffused through the swollen polymer.
ii. In the case of non-swellable polymers the termination of the function of the coating layer is achieved by the breaking of the inelastic coating layer due to the swelling of the core as the core is hydrated through the free surfaces and the pores that are created after the soluble compound of the coating layer is dissolved.

The duration of the function of the coating layer is depended on:
i. The composition of the coating layer and more specifically the polymer-soluble compound ratio. The polymer is usually added at a 1:1 to 9:1 ratio to the water-soluble compound.
ii. The thickness of the coating layer
iii. The kind of the polymer and the kind of the soluble compound
iv. In the case of non-swellable polymers the resistance of the coating layer is also depended on the presence and the percentage of plasticizers. The plasticizers are used in a percentage that ranges between 0-10% and increase the elasticity and consequently the endurance of the coating layer. This way the time period that the coating layer is functional can be controlled through another parameter, the percentage of the plasticizer. Plasticizers used in such formulations are polyethylene glycol, triethyl citrate, glycerol, 1,2 propylene glycol.

The coating layer is applied on the core by a compression process, after mixing the excipients that compose it. As a result the coating layer may contain classical excipients used in direct compression processes, such as glidants, lubricants, diluents and binders. In contrast with the formulation of the cores the coating layer may contain disintegrating agents (such as microcrystalline cellulose, pregelatinized starch, sodium starch glycollate and calcium carboxymethyl cellulose) in proportion between 0-5%, as long as these agents enhance the formation of pores through the polymer mass and do not affect the continuity of the coating layer during the early stages of the drug release.

The cores may also be film coated. Similarly to the coating layer, the coating material is functional for a determined period of time that does not exceed the first 4 hours of the drug release from the core. The film coating usually represents from 1.5 to 18%, by weight of the weight of the mini tablet.

The film coating material contains a polymer at a proportion that ranges between 10-80% of the dry mass of the coating material. The said polymer creates a film that covers the core, reducing the surface of the core that is initially available for the dissolution of the drug substance.

The delivery of the drug substance in the initial stages of the wetting of the coated mini tablets is through pores that are created by the dissolution of water soluble compounds that the coating film contains in a proportion that usually ranges from 20-50% by weight.

The polymers that can be used are:
i. Swellable polymers such as those recited above with respect to the formulation of the core.
ii. Non-swellable such as those recited above with respect to the formulation of the core.
iii. pH-dependent polymers that are insoluble in acidic environment (like the gastric fluids), while they dissolve in slightly acidic (pH 4.5-5.5), neutral or slightly basic pH (6.0-8.0). Such polymers are:
   a) Cellulose acetate phthalate, a polymer that dissolves at pH values over 6.4.
   b) Poly(butyl methacrylate, (2-dimethyl aminoethyl) methacrylate, methyl methacrylate) 1:2:1 copolymer, commercially available as Eudragit E®, that dissolves in pH values lower than 5.
   c) poly(ethyl acrylate, methyl methacrylate) 2:1 copolymer, commercially available as Eudragit 30D®, that dissolves in pH values of about 5.5.
   d) poly(methacrylic acid, methyl methacrylate) 1:1 copolymer, commercially available as Eudragit L®, that dissolves in pH values of about 6.7.
   e) poly(methacrylic acid, methyl methacrylate) 1:2 copolymer, commercially available as Eudragit S®, that dissolves in pH values of about 6.7.

The water-soluble compound may be the same as the ones recited above with respect to the water-soluble compounds of the coating layer.

The coating material may also contain classical excipients such as those recited above with respect to the formulation of the core, as well as plasticizers (such as those recited above with respect to the formulation of the coating layer), colorants (e.g. quinoline yellow, indigotine, sunset yellow), opacifiers (usually titanium dioxide), adhesive agents (such as low viscosity hydroxypropyl methyl cellulose, hydroxypropyl cellulose and polyvinylpyrrolidone), at a total proportion that ranges between 10-50% by weight of the total weight of the dry coating material.

Ethanol, acetone, water isopropyl alcohol, methylene chloride, chloroform or any other pharmaceutically suitable solvent may be used, as well mixtures of the said solvents, as long as can dissolve or uniformly disperse the constituents of the coating mixture. The solid content of the coating solution or dispersion typically ranges between 3-40% by weight. The dissolution or dispersion of the solid content of the coating material may be optimised by the use of polyethylene glycol in an amount from 0 to 10% by weight of the coating material.

In the case of the film coating the function of the coating is terminated at an optimal time period as said above. The mechanisms that cause the termination of the function of the film coating are:
i. In the case of coating films consisting of swellable polymers the function of the coating is terminated when the diffusion layer reaches the surface of the core. After that stage the surface of the core that was covered by the polymer layer can be hydrated and the drug substance can be diffused through the swollen polymer, similarly to the process recited above for the coating layers.
ii. In the case of coating films consisting of non-swellable polymers the function of the coating is terminated when the swelling of the core breaks the polymer layer, similarly to the process recited above for the coating layers. The diffusion of the soluble compound creates pores through the core can be hydrated and swell.
iii. In the case of pH-dependent polymer films the function of the coating is terminated through two potential mechanisms: firstly the same mechanism that occurs when the non-swelling polymers are enabled and secondly the change of the pH of the aqueous environment throughout the gastrointestinal track.

### PREFERRED EMBODIMENTS:

One preferred embodiment is a capsule containing an appropriate number of mini tablets, in a way that linearity between the strength and the total weight of the dosage form is achieved (1 to 6 mini tablets per capsule).

Each tablet comprises:
i. A functional core comprising Venlafaxine HCl, one or more gelling agents, one or more non-swelling agents, one or more conjugation agents and appropriate quantities of classical excipients
ii. A functional coating comprising an enteric film coating containing water-soluble compound.

### EXAMPLES: The following examples illustrate the present invention.

### 1) EXAMPLES ILLUSTRATING THE INVENTION MACROSCOPICALLY

- Example 1.1: a 0 or 00 size capsule containing 1-6 Venlafaxine 25mg coated mini-tablets
- Example 1.2: a 0 or 00 size capsule containing 1-4 Venlafaxine 37.5mg coated mini-tablets
- Example 1.3: a 0 or 00 size capsule containing 1-3 Venlafaxine 50mg coated mini-tablets
- Example 1.4: a 0 or 00 size capsule containing 1-2 Venlafaxine 75mg coated mini-tablets

### 2) EXAMPLES ILLUSTRATING THE CORE:

**Example 2.1:** the following formulation was prepared:

| **Ingredient** | **Venlafaxine 25 mg core** | **Venlafaxine 37.5 mg core** | **Venlafaxine 50 mg core** | **Ventafaxine 75 mg core** | **% in the core** |
|---|---|---|---|---|---|
| **Venlafaxine HCl (equivelant to 1:1.132 Venlafaxine base)** | 28,30 | 42,45 | 56,60 | 84,90 | 26,87 |
| **Sodium Lauryl Sulphate** | 7,37 | 11,06 | 14,75 | 22,12 | 7,00 |
| **Eudragit RS 100** | 7,07 | 10,61 | 14,15 | 21,22 | 6,72 |
| **Methocel K100 M** | 62,06 | 93,09 | 124,12 | 186,18 | 58,92 |
| **Magnesium stearate** | 0,53 | 0,79 | 1,05 | 1,58 | 0,50 |
| **Total** | 105,33 | 158,00 | 210,67 | 316,00 | 100,00 |

Manufacturing process: Venlafaxine HCl, Methocel K 100 M®, and SLS are sieved through a 30 mesh sieve and mixed for an appropriate time period until a uniform mixture is formed. This mixture comprises the internal phase of the formulation. Eudragit RS 100® is dissolved in acetone, preparing a wet granulation fluid. The constituents of the internal phase are wet granulated using the wet granulation fluid. The granular mixture is dried to constant weight in an oven at 40°C (the total content in solvents is estimated using the Loss on drying method as described in the European Pharmacopoeia 3^{rd} Edition and should be less than 1.5%).

The dry granule is mixed with the rest of the excipients in a drum mixer and the resulting mixture is pressed into biconvex tablets (almost spherical in shape) of appropriate mass relatively to the strength and hardness using a Killian® tabletting machine. For the Venlafaxine 25mg cores 5mm punches were used, for the 37.5 and 50mg cores 6mm punches were used, while for the 75mg cores 7mm punches were used. The mini-tablets are placed into 00-sized capsules.

**Example 2.2:** the following formulation was prepared:

| | | | | | |
|---|---|---|---|---|---|
| | | | | | |
| **Venlafaxine HCl (equivelant to 1:1.132 Venlafaxine base)** | 28,30 | 42,45 | 56,60 | 84,90 | 26,87 |
| **Sodium Lauryl Sulphate** | 5,00 | 7,50 | 10,00 | 15,00 | 4,75 |
| **Eudragit RS 100** | 7,07 | 10,61 | 14,15 | 21,22 | 6,72 |
| **Methocel K100 M** | 57,36 | 86,04 | 114,72 | 172,08 | 54,46 |
| **Kollidon SR** | 7,07 | 10,61 | 14,15 | 21,22 | 6,72 |
| **Magnesium stearate** | 0,53 | 0,79 | 1,05 | 1,58 | 0,50 |
| **Total** | 105,33 | 158,00 | 210,67 | 316,00 | 100,00 |

Manufacturing process: the same manufacturing process as the one cited above with respect to the core of example 2.1. Kollidon SR® is a commercial name for polyvinylpyrrolidone acetate and it was added in the internal phase.

**Example 2.3**: the following formulation was prepared:

| **Ingredient** | **Venlafaxine 25 mg core** | **Venlafaxine 37.5 mg core** | **Venlafaxine 50 mg core** | **Venlafaxine 75 mg core** | **% in the core** |
|---|---|---|---|---|---|
| **Venlafaxine HCl (equivelant to 1:1.132 Venlafaxine base)** | 28,30 | 42,45 | 56,60 | 84,90 | 26,87 |
| **Sodium Lauryl Sulphate** | 6,32 | 9,48 | 12,64 | 18,96 | 6,00 |
| **Eudragit RS 100** | 7,07 | 10,61 | 14,15 | 21,22 | 6,72 |
| **Methocel K100** M | 56,04 | 84,06 | 112,08 | 168,12 | 53,20 |
| **Kollidon SR** | 7,07 | 10,61 | 14,15 | 21,22 | 6,72 |
| **Magnesium stearate** | 0,53 | 0,79 | 1,05 | 1,58 | 0,50 |
| **Total** | 105,33 | 158,00 | 210,67 | 316,00 | 100,00 |

Manufacturing process: the same manufacturing process as the one cited above with respect to the core of example 2.1. Kollidon SR® is a commercial name for polyvinylpyrrolidone acetate and it was added in the internal phase.

**Example 2.4**: the following formulation was prepared:

| **Ingredient** | **Venlafaxine 25 mg core** | **Venlafaxine 37.5 mg core** | **Venlafaxine 50 mg core** | **Venlafaxine 75 mg core** | **% in the core** |
|---|---|---|---|---|---|
| **Venlafaxine HCl (equivelant to 1:1.132 Venlafaxine base)** | 28,30 | 42,45 | 56,60 | 84,90 | 26,87 |
| **Sodium Lauryl Sulphate** | 8,43 | 12,64 | 16,85 | 25,28 | 8,00 |
| **Eudragit RS 100** | 7,07 | 10,61 | 14,15 | 21,22 | 6,72 |
| **Methocel K100 M** | 53,93 | 80,90 | 107,87 | 161,80 | 51,20 |
| **Kollidon SR** | 7,07 | 10,61 | 14,15 | 21,22 | 6,72 |
| **Magnesium stearate** | 0,53 | 0,79 | 1,05 | 1,58 | 0,50 |
| **Total** | 105,33 | 158,00 | 210,67 | 316,00 | 100,00 |

Manufacturing process: the same manufacturing process as the one cited above with respect to the core of example 2.1. Kollidon SR® is a commercial name for polyvinylpyrrolidone acetate and it was added in the internal phase.

**Example 2.5:** the following formulation was prepared:

| **Ingredient** | **Venlafaxine 25 mg core** | **Venlafaxine 37.5 mg core** | **Venlafaxine 50 mg core** | **Venlafaxine 75 mg core** | **% in the core** |
|---|---|---|---|---|---|
| **Venlafaxine HCl (equivelant to 1:1.132 Venlafaxine base)** | 28,30 | 42,45 | 56,60 | 84,90 | 26,87 |
| **Sodium Lauryl Sulphate** | 8,43 | 12,64 | 16,85 | 25,28 | 8,00 |
| **Eudragit RS 100** | 14,15 | 21,22 | 28,29 | 42,44 | 13,43 |
| **Methocel K100 M** | 35,57 | 53,36 | 71,15 | 106,72 | 33,77 |
| **Kollidon SR** | 14,15 | 21,22 | 28,29 | 42,44 | 13,43 |
| **Magnesium stearate** | 0,53 | 0,79 | 1,05 | 1,58 | 0,50 |
| **Talc** | 4,21 | 6,32 | 8,43 | 12,64 | 4,00 |
| **Total** | 105,33 | 158,00 | 210,67 | 316,00 | 100,00 |

Manufacturing process: the same manufacturing process as the one cited above with respect to the core of example 2.1. Kollidon SR® is a commercial name for polyvinylpyrrolidone acetate and it was added in the internal phase.

**Example 2.6:** the following formulation was prepared:

| **Ingredient** | **Venlafaxine 25 mg core** | **Venlafaxine 37.5 mg core** | **Venlafaxine 50 mg core** | **Venlafaxine 75 mg core** | **% in the core** |
|---|---|---|---|---|---|
| **Venlafaxine HCl (equivelant to 1:1.132 Venlafaxine base)** | 28,30 | 42,45 | 56,60 | 84,90 | 26,87 |
| HPC | 5,00 | 7,50 | 10,00 | 15,00 | 4,75 |
| EudragitRS 100 | 7,07 | 10,61 | 14,15 | 21,22 | 6,72 |
| Methocel K100 M | 57,36 | 86,04 | 114,72 | 172,08 | 54,46 |
| Kollidon SR | 7,07 | 10,61 | 14,15 | 21,22 | 6,72 |
| Magnesium stearate | 0,53 | 0,79 | 1,05 | 1,58 | 0,50 |
| Total | 105,33 | 158,00 | 210,67 | 316,00 | 100,00 |

Manufacturing process: the same manufacturing process as the one cited above with respect to the core of example 2.1. Kollidon SR® is a commercial name for polyvinylpyrrolidone acetate and it was added in the internal phase.

The release profiles of the above formulations were tested using a dissolution apparatus with paddles at 100 rpm using 500ml of a pH 1.2 solution for the first two hours and 1000ml of phosphate buffer solution for the rest of the test (total duration 24 h). The results of the dissolution tests are presented in the following table 1 and figure 2:

| **Time** | **Table1: dissolution tests concerning the cores described in examples 2.1 to 2.6 (% Dissolved)** | | | | | |
|---|---|---|---|---|---|---|
| | **Core of example 2.1** | **Core of example 2.2** | **Core of example 2.3** | **Core of example 2.4** | **Core of example 2.5** | **Core of example 2.6** |
| **1** | 17,2 | 25,5 | 19,7 | 19,1 | 19,2 | 27,5 |
| **2** | 32,1 | 36,0 | 28,9 | 26,6 | 27,8 | 37,1 |
| **4** | 41,2 | 44,0 | 35,9 | 33,2 | 33,5 | 46,1 |
| **6** | 49,7 | 52,2 | 43,2 | 40,1 | 39,8 | 54,4 |
| **8** | 63,5 | 66,0 | 53,9 | 51,7 | 48,0 | 67,9 |
| **10** | 72,7 | 75,0 | 63,4 | 60,2 | 55,6 | 77,7 |
| **12** | 79,5 | 81,8 | 71,1 | 68,2 | 64,5 | 85,8 |
| **16** | 84,6 | 86,2 | 74,5 | 71,7 | 66,1 | 87,8 |
| **20** | 89,9 | 91,3 | 80,7 | 76,0 | 71,9 | 93,5 |
| **24** | 95,6 | 100,0 | 83,9 | 80,7 | 76,3 | 102,0 |

Similar results were noticed when the other strengths were tested for the release of the drug substance, as well as when combinations of the above cores were tested.

The cores containing a conjugation agent exhibited lower initial release of the drug substance Venlafaxine HCl, in a degree that ranges between 3-10%.

There is an optimal ratio between the quantities of the swelling polymer, the non-swelling polymer and the conjugation agent. At this ratio the initial release of Venlafaxine HCl from the formulation is reduced, while the drug substance is quantitatively released from the core at the end of the test (set at 24 hours).

### 3) EXAMPLES ILLUSTRATING THE COATING LAYER:

The following examples of the coating layer are applied using the core described in example 2.1 as a model core, so that the effect of the coating layer on the formulation can be evaluated. The 75mg core was enabled as a worst case as it is the core with the biggest surface.

Example 3.1: based on the core described in example 2.1 and the following formulation was prepared for the coating layer:

| Example 3.1 | |
|---|---|
| Coating Layer Constituents | % |
| Cellulose Acetate Propionate | 99,0 |
| Magnesium Stearate | 1,0 |

The constituents of the coating layer are mixed until a uniform powder mixture is prepared. Then the coating layer is applied by compression on the precompressed core. For the two layer and the three layer tablets the coating layer is applied using the same punches as the ones used for the compression of the core. In the case that the perimeter and one side of the core are coated the punch used for the application of the coating layer is of bigger diameter (usually 1 to 4mm larger than the diameter of the core). Two levels of the thickness of the coating layer were tested, 1.0 and 2.0 mm, as for the effect of the coating layer on the dissolution profile of complex tablets.

Example 3.2: based on the core described in example 2.1 and the following formulation was prepared for the coating layer:

| Example 3.2 | |
|---|---|
| Coating Layer Constituents | % |
| Methocel E 50LV | 99 |
| Magnesium Stearate | 1 |

Manufacturing process: the same manufacturing process as the one recited above with respect to the coating layers of example 3.1.

Example 3.3: based on the core described in example 2.1 and the following formulation was prepared for the coating layer:

| Example 3.3 | |
|---|---|
| Coating Layer Constituents | % |
| POLYOX 900000 | 99 |
| Magnesium Stearate | 1 |

Manufacturing process: the same manufacturing process as the one recited above with respect to the coating layers of example 3.1.

Example 3.4: based on the core described in example 2.1 and the following formulation was prepared for the coating layer:

| Example 3.4 | |
|---|---|
| Cellulose Acetate Propionate | 79,0 |
| PVP | 20,0 |
| Magnesium Stearate | 1,0 |

Manufacturing process: the same manufacturing process as the one recited above with respect to the coating layers of example 3.1.

Example 3.5: based on the core described in example 2.1 and the following formulation was prepared for the coating layer:

| Example 3.5 | |
|---|---|
| Cellulose Acetate Propionate | 74,0 |
| PVP | 20,0 |
| PEG | 5,0 |
| Magnesium Stearate | 1,0 |

Manufacturing process: the same manufacturing process as the one recited above with respect to the coating layers of example 3.1.

Example 3.6: based on the core described in example 2.1 and the following formulation was prepared for the coating layer:

| Example 3.6 | |
|---|---|
| Cellulose Acetate Propionate | 71,5 |
| PVP | 17,5 |
| PEG | 10,0 |
| Magnesium Stearate | 1,0 |

Manufacturing process: the same manufacturing process as the one recited above with respect to the coating layers of example 3.1.

Example 3.7: based on the core described in example 2.1 and the following formulation was prepared for the coating layer:

| Example 3.7 | |
|---|---|
| Methocel E 50LV | 79,0 |
| PVP | 20,0 |
| Magnesium Stearate | 1,0 |

Manufacturing process: the same manufacturing process as the one recited above with respect to the coating layers of example 3.1.

Example 3.8: based on the core described in example 2.1 and the following formulation was prepared for the coating layer:

| Example 3.8 | |
|---|---|
| Methocel E 50LV | 79,0 |
| Lactose | 20,0 |
| Magnesium Stearate | 1,0 |

Manufacturing process: the same manufacturing process as the one recited above with respect to the coating layers of example 3.1.

The release profiles of the above complex systems were tested using a dissolution apparatus with paddles at 100 rpm using 500ml of a pH 1.2 solution for the first two hours and 1000ml of phosphate buffer solution for the rest of the test (total duration 24 h).

The results of the dissolution tests performed for the most typical of the above systems are presented in the following tables:

| Table 2: dissolution profile using the coating layer described in example 3.1 | | | | | | | | |
|---|---|---|---|---|---|---|---|---|
| Coating layer | Kind of complex system | Thickness of coating layer | %Release of Venlafaxne HCl | | | | | |
| | | | 1h | 2h | 4h | 10h | 16h | 24h |
| Example 3.1 | Two layer tablets | 1mm | 15,0 | 28,7 | 38,1 | 70,9 | 82,3 | 96,1 |
| | | 2mm | 14,2 | 28,0 | 37,6 | 70,1 | 81,4 | 95,4 |
| | Three layer tablets | 1mm | 10,9 | 26,3 | 33,4 | 68,9 | 80,5 | 93,4 |
| | | 2mm | 11,1 | 26,7 | 32,8 | 67,7 | 80,2 | 93,1 |
| | Coating of the perimeter and one side of the core | 1mm | 5,4 | 23,7 | 31,8 | 67,5 | 81,0 | 94,2 |
| | | 2mm | 5,2 | 22,3 | 30,7 | 66,7 | 80,2 | 93,7 |

| Table 3: dissolution profile using the coating layer described in example 3.2 | | | | | | | | |
|---|---|---|---|---|---|---|---|---|
| Coating layer | Kind of complex system | Thickness of coating layer | %Release of Venlafaxine HCl | | | | | |
| | | | 1h | 2h | 4h | 10h | 16h | 24h |
| Example 3.2 | Two layer tablets | 1mm | 14,5 | 26,5 | 35,3 | 70,5 | 83,6 | 97,1 |
| | | 2mm | 13,9 | 26,9 | 34,9 | 70,1 | 71,7 | 98,1 |
| | Three layer tablets | 1mm | 9,8 | 25,3 | 32,7 | 68,9 | 72,3 | 99,1 |
| | | 2mm | 9,6 | 24,9 | 30,1 | 67,4 | 72,1 | 99,2 |
| | Coating of the perimeter and one side of the core | 1mm | 5,5 | 20,4 | 29,4 | 65,3 | 78,9 | 93,7 |
| | | 2mm | 5,2 | 18,0 | 25,3 | 59,8 | 72,3 | 91,7 |

| Table 4: dissolution profile using the coating layer described in example 3.4 | | | | | | | | |
|---|---|---|---|---|---|---|---|---|
| Coating layer | Kind of complex system | Thickness of coating layer | %Release of Venlafaxine HCl | | | | | |
| | | | 1h | 2h | 4h | 10h | 16h | 24h |
| Example 3.4 | Two layer tablets | 1mm | 16,2 | 30,1 | 38,7 | 72,4 | 85,1 | 96,7 |
| | | 2mm | 16,1 | 29,4 | 38,4 | 71,0 | 84,2 | 96,4 |
| | Three layer tablets | 1mm | 12,1 | 28,3 | 35,6 | 70,3 | 85,6 | 97,8 |
| | | 2mm | 10,1 | 27,2 | 33,9 | 67,9 | 84,7 | 96,8 |
| | Coating of the perimeter and one side of the core | 1 mm | 4,9 | 21,3 | 28,4 | 64,3 | 79,8 | 94,3 |
| | | 2mm | 4,6 | 18,9 | 27,6 | 63,2 | 76,6 | 92,5 |

| Table 5: dissolution profile using the coating layer described in example 3.6 | | | | | | | | |
|---|---|---|---|---|---|---|---|---|
| Coating layer | Kind of complex system | Thickness of coating layer | %Release of Venlafaxine HCl | | | | | |
| | | | 1h | 2h | 4h | 10h | 16h | 24h |
| Example 3.6 | Two layer tablets | 1mm | 15,9 | 24,6 | 36,2 | 70,9 | 84,2 | 95,8 |
| | | 2mm | 16,1 | 24,3 | 31,2 | 68,4 | 82,1 | 96,7 |
| | Three layer tablets | 1mm | 12,3 | 22,1 | 34,1 | 70,2 | 82,6 | 96,8 |
| | | 2mm | 10,1 | 22,3 | 28,9 | 65,2 | 79,8 | 92,3 |
| | Coating of the perimeter and one side of the core | 1 mm | 5,2 | 16,4 | 24,3 | 59,8 | 74,5 | 89,1 |
| | | 2mm | 4,9 | 16,2 | 23,2 | 57,9 | 73,2 | 88,1 |

| Table 6: dissolution profile using the coating layer described in example 3.8 | | | | | | | | |
|---|---|---|---|---|---|---|---|---|
| Coating layer | Kind of complex system | Thickness of coating layer | %Release of Venlafaxine HCl | | | | | |
| | | | 1h | 2h | 4h | 10h | 16h | 24h |
| Example 3.8 | Two layer tablets | 1mm | 16,2 | 29,7 | 39,5 | 79,0 | 93,6 | 98,9 |
| | | 2mm | 15,6 | 30,1 | 39,1 | 78,5 | 80,3 | 99,1 |
| | Three layer tablets | 1mm | 11,0 | 28,3 | 36,6 | 77,2 | 81,0 | 97,8 |
| | | 2mm | 10,8 | 27,9 | 33,7 | 75,5 | 80,8 | 96,3 |
| | Coating of the perimeter and one side of the core | 1mm | 6,2 | 22,8 | 32,9 | 73,1 | 88,4 | 99,7 |
| | | 2mm | 5,8 | 20,2 | 28,3 | 67,0 | 81,0 | 98,1 |

The above results show that the said complex systems can be applied with great flexibility and cause an extensive reduction of the initial drug release. This reduction ranges from 2 to 13.5%, while the duration of the function of the coating layer can also be controlled and manipulated.

### 4) EXAMPLES ILLUSTRATING THE COATING FILM:

The following examples of the coating films are applied on the core described in example 2.1 as a model core, so that the effect of the coating film on the formulation can be evaluated. The 75mg core was enabled as a worst case as it is the core with the biggest surface.

Example 4.1: based on the core described in example 2.1 and the following formulation was prepared for the coating film:

| Example 4.1 | |
|---|---|
| Coating Film Constituents | % |
| Eudragit RS | 50,0 |
| PEG | 5,0 |
| Talc | 15,0 |
| Lactose | 20,0 |
| Magnesium stearate | 10,0 |
| Solvents: Acetone, Acetone:Ethanol 1:1 | |

Manufacturing process: The constituents of the coating film are dispersed in the solvent mixture preparing a homogeneous dispersion of 5-15% solid content. Then the coating film is spray-coated on the cores that were preheated at 70°C. The coating process was completed when the film coating of each core reached a weight of 7-10% of the weight of the core. The film-coated cores were dried for 2hours at 40°C.

Example 4.2: based on the core described in example 2.1 and the following formulation was prepared for the coating film:

| Example 4.2 | |
|---|---|
| Coating Film Constituents | % |
| Ethyl Cellulose | 30,0 |
| HPMC 50cp | 20,0 |
| PEG | 5,0 |
| Talc | 15,0 |
| PVP | 20,0 |
| Magnesium stearate | 10,0 |
| Solvents: Acetone:Isopropanol 1:1 | |

Manufacturing process: the same manufacturing process as the one recited above with respect to the coating film of example 4.1.

Example 4.3: based on the core described in example 2.1 and the following formulation was prepared for the coating film:

| Example 4.3 | |
|---|---|
| Coating Film Constituents | % |
| Cellulose Acetate Propionate | 30,0 |
| HPMC 50cp | 10,0 |
| PEG | 15,0 |
| Talc | 15,0 |
| PVP | 20,0 |
| Magnesium stearate | 10,0 |
| Solvents: Acetone:Isopropanol 1:1 | |

Manufacturing process: the same manufacturing process as the one recited above with respect to the coating film of example 4.1.

Example 4.4: based on the core described in example 2.1 and the following formulation was prepared for the coating film:

| Example 4.4 | |
|---|---|
| Coating Film Constituents | % |
| Cellulose Acetate Phthalate | 30,0 |
| Ethyl Cellulose | 10,0 |
| PEG | 15,0 |
| Talc | 15,0 |
| PVP | 20,0 |
| Magnesium stearate | 10,0 |
| Solvents: Acetone:Ethanol 1:1, Acetone, Acetone:H20 97:3 | |

Manufacturing process: Manufacturing process: the same manufacturing process as the one recited above with respect to the coating film of example 4.1.

Example 4.5: based on the core described in example 2.1 and the following formulation was prepared for the coating film:

| Example 4.5 | |
|---|---|
| Coating Film Constituents | % |
| Kollicoat SR 30 D | 60,0 |
| Propylene Glycol | 12,5 |
| Talc | 7,5 |
| PVP | 10,0 |
| Magnesium stearate | 10,0 |
| Water | |

Manufacturing process: the same manufacturing process as the one recited above with respect to the coating film of example 4.1.

Example 4.6: based on the core described in example 2.1 and the following formulation was prepared for the coating film:

| Example 4.6 | |
|---|---|
| Coating Film Constituents | % |
| Eudragit L | 12,5 |
| Eudragit S | 37,5 |
| Dibutyl sebacate | 5,0 |
| Talc | 15,0 |
| Lactose | 20,0 |
| Magnesium stearate | 10,0 |
| Solvents: Acetone:Isopropanol 1:1 | |

Manufacturing process: the same manufacturing process as the one recited above with respect to the coating film of example 4. 1.

Example 4.7: based on the core described in example 2.1 and the following formulation was prepared for the coating film:

| Example 4.7 | |
|---|---|
| Coating Film Constituents | % |
| Eudragit L | 37,5 |
| Eudragit S | 12,5 |
| Dibutyl sebacate | 5,0 |
| Talc | 15,0 |
| PVP | 20,0 |
| Magnesium stearate | 10,0 |
| Solvents: Acetone:Isopropanol 1:1 | |

Manufacturing process: the same manufacturing process as the one recited above with respect to the coating film of example 4.1.

| Table 7: dissolution profiles of the film coated cores (75mg core as described in example 2.1) | | | | | | | |
|---|---|---|---|---|---|---|---|
| Kind of coating film | Weight of the coating film as %of the core weight | %Release of Venlafaxne HCl | | | | | |
| | | 1h | 2h | 4h | 10h | 16h | 24h |
| Example 4.1 | 4% | 9,9 | 26,8 | 33,4 | 71,3 | 83,8 | 95,2 |
| | 8% | 6,5 | 24,1 | 34,7 | 75,6 | 88,9 | 102,1 |
| Example 4.2 | 4% | 13,8 | 26,6 | 34,5 | 69,4 | 71,0 | 97,1 |
| | 8% | 8,0 | 24,3 | 35,0 | 76,1 | 85,6 | 100,1 |
| Example 4.3 | 4% | 12,2 | 29,5 | 33,9 | 71,5 | 83,4 | 95,6 |
| | 8% | 5,6 | 18,5 | 26,4 | 66,0 | 84,5 | 100,4 |
| Example 4.4 | 4% | 10,4 | 20,9 | 28,9 | 64,8 | 79,4 | 94,0 |
| | 8% | 4,6 | 18,9 | 27,6 | 63,2 | 76,6 | 92,5 |
| Example 4.5 | 4% | 12,3 | 27,1 | 35,9 | 73,3 | 87,2 | 103,9 |
| | 8% | 8,9 | 23,7 | 32,6 | 69,3 | 83,1 | 98,8 |
| Example 4.6 | 4% | 12,6 | 29,4 | 37,0 | 73,1 | 89,0 | 101,7 |
| | 8% | 8,7 | 26,8 | 34,5 | 72,0 | 85,1 | 102,3 |
| Example 4.7 | 4% | 15,6 | 28,9 | 37,2 | 69,5 | 82,6 | 97,4 |
| | 8% | 7,6 | 25,1 | 32,8 | 66,9 | 81,2 | 95,9 |

The above results show that the coating films can be applied with great flexibility and cause an extensive reduction of the initial drug release. This reduction ranges from about 3 to about 13%, while the duration of the function of the film coating can also be controlled and manipulated.

## Claims

1. A pharmaceutical dosage form comprising an extended release formulation of the water-soluble drug substance Venlafaxine HCl, comprising a hard gelatin capsule containing a therapeutically effective number of mini tablets comprising a functional core prepared by wet granulation, drying and compression process and a functional coating layer or coating film, so that the initial rapid release of the drug substance from the cores is limited.

2. A pharmaceutical dosage form according to claim 1 wherein the cores of the mini tablets are composed of 10-40% by weight of Venlafaxine HCl, 40-80% by weight of a gelling agent, 30-60% by weight of a non-swelling agent, 2-12% by weight of a conjugation agent and 1-30% by weight of classical excipients with the exception of excipients that exhibit disintegrating properties.

3. A pharmaceutical dosage form according to claim 2, wherein the gelling agent is a polymer selected from the group consisting of hydroxypropylmethylcellulose, hydroxypropylcellulose, hydroxycellulose phthalate, poly(ethyleneoxide), polylactic acid, xanthan gum, alginates, sodium and calcium carboxymethylcellulose, carragheen, carbomer, carbopol for oral use, methylhydroxyethylcellulose, propylhydroxyethylcellulose, polyhema, methylcellulose and alginates.

4. A pharmaceutical dosage form according to claim 2 wherein the non-swelling polymer is selected from a group comprising ethyl cellulose, cellulose acetate propionate, cellulose acetate, poly(ethyl acrylate, methyl methacrylate, trimethylammonioethyl methacrylate chloride) 1:2:0.1, commerced as Eudragit RS 100® poly(ethyl acrylate, methyl methacrylate, trimethylammonioethyl methacrylate chloride) 1:2:0.2 copolymer, commercially available as Eudragit RL®, polyvinylpyrrolidone acetate, polyvinyl chloride, polyvinyl acetate or polyethylene.

5. A pharmaceutical dosage form according to claim 2 wherein the polymers of the core are conjugated by a pharmaceutically accepted conjugation agent, such as sodium lauryl sulphate, sodium docusate, sodium cetostearyl sulphate and triethanolamine lauryl sulphate, that causes the decrease on the swelling properties of the core.

6. A pharmaceutical dosage form as defined in claim 1, wherein the cores are partially coated by a functional coating layer, covering one or two surfaces of the core, or one surface and the perimeter of the core and the thickness of the coating layer ranging between 3-30% of the diameter of the core.

7. A pharmaceutical dosage form as defined in claim 6, wherein the functional coating layer is comprised of a polymer and a water soluble compound, wherein said polymer and said water soluble compound are present in a weight ratio of 1:1 to 9:1.

8. A pharmaceutical dosage form as defined in claim 6, wherein the polymer is either selected from the group consisting of swellable polymers as the ones cited above in claim 3, or from the group consisting of non-swellable polymers, as the ones cited above in claim 4.

9. A pharmaceutical dosage form as defined in claim 6, wherein the water soluble compound is selected either from the group of water soluble salts, such as sodium chloride, sodium bicarbonate or the group of low relative molecular mass organic solid excipients, such as mannitol, lactose, sucrose, sorbitol or citric acid or from the group of water soluble polymers such as polyvinylpyrrolidone, polyvinyl alcohol or low viscosity hydroxypropylmethyl cellulose.

10. A pharmaceutical dosage form as defined in claim 1 wherein the cores are film coated by a functional coating film, that represent 1.5 to 18% by weight of the weight of the core, applied to a sufficient thickness to reduce the initial release of the drug substance from the formulation.

11. A pharmaceutical dosage form as defined in claim 10, wherein the functional coating film is comprised of a polymer in a proportion of 10-80% of the dry coating material and a water soluble compound, in a proportion of 20-50% of the dry coating material.

12. A pharmaceutical dosage form as defined in claim 11, wherein the polymer is selected either from the group consisting of swellable polymers such as the ones cited in claim 3, or from the group consisting of non-swellable polymers such as the ones cited in claim 4, or from the group of pH-depended polymers that are insoluble in acidic environments while they soften or dissolve in neutral or basic environments, such as cellulose acetate phthalate, poly(butyl methacrylate, (2-dimethyl aminoethyl) methacrylate, methyl methacrylate) 1:2:1 copolymer, commercially available as Eudragit E®, poly(ethyl acrylate, methyl methacrylate) 2:1 copolymer, commercially available as Eudragit 30D®, poly(methacrylic acid, methyl methacrylate) 1:1 copolymer, commercially available as Eudragit L®, poly(methacrylic acid, methyl methacrylate) 1:2 copolymer, commercially available as Eudragit S®.

13. A pharmaceutical dosage form as defined in claim 11, wherein the water-soluble compound is selected from the groups recited above in claim 9.

14. A pharmaceutical dosage form as defined in claim 1 wherein the coating layer or the coating film further comprises a pharmaceutically accepted plasticizer.

15. A pharmaceutical dosage form as defined in claim 1, wherein the coating layer further comprises classical excipients selected from the group of binders, diluents, glidants, lubricants, adhesive agents, opacifiers and colourants.

16. A pharmaceutical dosage form as defined in claim 1, wherein the coating film further comprises classical excipients selected from the groups of and colourants.

17. A pharmaceutical dosage form as defined in claim 1, wherein the coating film is applied from a solution or dispersion of said polymer and said water soluble compound in a pharmaceutically acceptable solvent or mixture of pharmaceutically acceptable solvents where the selected constituents of the coating film can be uniformly dissolved or dispersed.

18. A pharmaceutical dosage form as defined in claim 2, wherein the drug substance the gelling agent, the non-swellable polymer and the conjugation agent are wet granulated using a pharmaceutically acceptable solvent or mixture of solvents.

19. A pharmaceutical dosage form as defined in claim 1, wherein the capsule comprises one to six of said mini tablets, each one containing 25 to 75 mg of the drug substance.

20. A pharmaceutical dosage form as defined in claim 1, wherein a linearity between the total weight of the mini tablets and the strength of the dosage form is achieved.

21. A pharmaceutical dosage form as defined in claim 1, wherein the dose may be divided by reducing the number of tablets in each capsule.

22. A pharmaceutical dosage form as defined in claim 1, comprising an extended release formulation for once daily administration, which comprises mini tablets partially or totally coated by a coating layer or coating film that is functional only during the first 2-4 hours of the drug release.

23. A method of preparing a drug delivery system for Venlafaxine, which comprises: a) preparing the cores containing Venlafaxine HCL according to claim 5 by a wet granulation, drying and compression process, b) applying a functional coating layer on the cores according to claim 9, using a direct compression process or applying a functional coating film on the cores using a spraying process, c) encapsulating the mini tablets by using an appropriate encapsulating device.

## Patentansprüche

1. Pharmazeutische Arzneiform, die eine Formulierung mit verlängerter Freisetzung des wasserlöslichen Wirkstoffs Venlafaxine HCl umfasst, die eine harte Gelatinekapsel umfasst, die eine therapeutisch wirksame Anzahl von Minitabletten enthält, die einem funktionellen Kern umfassen, der in einen Feuchtgranulierungs-, Trocknungs- und Kompressionsvorgang hergestellt wurde, und eine funktionelle Überzugsschicht oder einen Überzugsfilm umfasst, so dass die anfängliche schnelle Freisetzung des Wirkstoffs aus den Kernen begrenzt ist.

2. Pharmazeutische Arzneiform nach Anspruch 1, wobei die Kerne der Minitabletten aus 10 bis 40 Gew.-% Venlafaxine HCl, 40 bis 80 Gew.-% eines Geliermittels, 30 bis 60 Gew.-% eines nicht quellenden Mittels, 2 bis 12 Gew.-% eines Konjugationsmittels und 1 bis 30 Gew.-% eines klassischen Exzipienten bestehen, wobei Exzipienten ausgenommen sind, die zersetzende Eigenschaften aufweisen.

3. Pharmazeutische Arzneiform nach Anspruch 2, wobei das Geliermittel ein Polymer ist, ausgewählt aus der Gruppe aus Hydroxypropylmethylcellulose, Hydroxypropylcellulose, Hydroxycellulosephthalat, Poly(ethylenoxid), Polymilchsäure, Xanthangummi, Alginaten, Natrium- und Calciumcarboxymethylcellulose, Carrageen, Carbomer, Carbopol zur oralen Verwendung, Methylhydroxyethylcellulose, Propylhydroxyethylcellulose, Polyhema, Methylcellulose und Alginaten.

4. Pharmazeutische Arzneiform nach Anspruch 2, wobei das nicht quellende Polymer ausgewählt ist aus der Gruppe umfassend Ethylcellulose, Celluloseacetatpropionat, Celluloseacetat, Poly(ethylacrylat-Methylmethacrylat-Trimethylammonioethylmethacrylatchlorid) 1:2:0,1, vertrieben als Eudragit RS 100®, Poly(ethylacrylat-Methylmethacrylat-Trimethylammonioethylmethacrylatchlorid-Copolymer) 1:2:0,2, im Handel erhältlich als Eudragit RL®, Polyvinylpyrrolidonacetat, Polyvinylchlorid, Polyvinylacetat oder Polyethylen.

5. Pharmazeutische Arzneiform nach Anspruch 2, wobei die Polymere des Kerns durch ein pharmazeutisch akzeptiertes Konjugationsmittel, wie etwa Natriumlaurylsulfat, Natriumdocusat, Natriumcetostearylsulfat und Triethanolaminlaurylsulfat konjugiert werden, das zur Abnahme der Quelleigenschaften des Kerns führt.

6. Pharmazeutische Arzneiform, wie in Anspruch 1 definiert, wobei die Kerne teilweise mit einer funktionellen Überzugsschicht beschichtet sind, die eine oder zwei Oberflächen des Kerns bedeckt, oder eine Oberfläche und den Umfang des Kerns und wobei die Dicke der Überzugsschicht zwischen 3 und 30 % des Kerndurchmessers liegt.

7. Pharmazeutische Arzneiform wie in Anspruch 6 definiert, wobei die funktionelle Überzugsschicht aus einem Polymer und einer wasserlöslichen Verbindung besteht, wobei das Polymer und die wasserlösliche Verbindung in einem Gewichtsverhältnis von 1:1 bis 9:1 vorhanden sind.

8. Pharmazeutische Arzneiform wie in Anspruch 6 definiert, wobei das Polymer entweder aus der Gruppe ausgewählt ist, bestehend aus quellbaren Polymeren wie denjenigen, die oben in Anspruch 3 zitiert wurden, oder aus der Gruppe, bestehend aus nicht quellbaren Polymeren, wie denjenigen, die oben in Anspruch 4 zitiert wurden.

9. Pharmazeutische Arzneiform wie in Anspruch 6 definiert, wobei die wasserlösliche Verbindung entweder aus der Gruppe wasserlöslicher Salze ausgewählt ist, wie etwa Natriumchlorid, Natriumbicarbonat oder aus der Gruppe organischer fester Exzipienten mit relativ geringer Molekularmasse, wie etwa Mannitol, Lactose, Saccharose, Sorbitol oder Zitronensäure oder aus der Gruppe der wasserlöslichen Polymere, wie etwa Polyvinylpyrrolidon, Polyvinylalkohol oder niederviskoser Hydroxypropylmethylcellulose.

10. Pharmazeutische Arzneiform wie in Anspruch 1 definiert, wobei die Kerne mit einem Überzugsfilm beschichtet sind, der 1,5 bis 18 Gew.-% des Gewichts des Kerns darstellt, der in ausreichender Dicke aufgebracht ist, um die anfängliche Freisetzung des Wirkstoffs aus der Formulierung zu reduzieren.

11. Pharmazeutische Arzneiform wie in Anspruch 10 definiert, wobei der funktionelle Überzugsfilm aus einem Polymer in einem Verhältnis von 10 bis 80 % des trockenen Überzugsmaterials und einer wasserlöslichen Verbindung in einem Verhältnis von 20 bis 50 % des trockenen Überzugsmaterials besteht.

12. Pharmazeutische Arzneiform wie in Anspruch 11 definiert, wobei das Polymer entweder aus der Gruppe ausgewählt ist, bestehend aus quellbaren Polymeren, wie diejenigen, die in Anspruch 3 zitiert wurden, oder aus der Gruppe, bestehend aus nicht quellbaren Polymeren, wie denjenigen, die in Anspruch 4 zitiert wurden, oder aus der Gruppe der pH-abhängigen Polymere, die in sauren Milieus unlöslich sind, während sie in neutralen oder basischen Milieus aufweichen oder sich lösen, wie etwa Celluloseacetatphthalat, Poly(butylmethacrylat-(2-Dimethylaminoethyl)methacrylat-Methylmethacrylat-Copolymer) 1:2:1, im Handel erhältlich als Eudragit E®, Poly(ethylacrylat-Methylmethacrylat-Copolymer) 2:1, im Handel erhältlich als Eudragit 30D®, Poly(methacrylsäure-Methylmethacrylat-Copolymer) 1:1, im Handel erhältlich als Eudragit L®, Poly(methacrylsäure-Methylmethacrylat-Copolymer) 1:2, im Handel erhältlich als Eudragit S®.

13. Pharmazeutische Arzneiform wie in Anspruch 11 definiert, wobei die wasserlösliche Verbindung aus den Gruppen ausgewählt ist, wie in Anspruch 9 oben angeführt.

14. Pharmazeutische Arzneiform wie in Anspruch 1 definiert, wobei die Überzugsschicht oder der Überzugsfilm ferner einen pharmazeutisch akzeptablen Weichmacher umfassen.

15. Pharmazeutische Arzneiform wie in Anspruch 1 definiert, wobei die Überzugsschicht ferner klassische Exzipienten umfasst, ausgewählt aus der Gruppe von Bindemitteln, Verdünnungsmitteln, Fließhilfsstoffen, Gleitmitteln, Haftmitteln, Trübungsmitteln und Farbstoffen.

16. Pharmazeutische Arzneiform wie in Anspruch 1 definiert, wobei der Überzugsfilm ferner klassische Exzipienten umfasst, die aus der Gruppe der Farbstoffe ausgewählt sind.

17. Pharmazeutische Arzneiform wie in Anspruch 1 definiert, wobei der Überzugsfilm aus einer Lösung oder Dispersion des Polymers und der wasserlöslichen Verbindung in einem pharmazeutisch akzeptablen Lösungsmittel oder einer Mischung aus pharmazeutisch akzeptablen Lösungsmitteln aufgetragen wird, worin die ausgewählten Bestandteile des Überzugsfilms gleichmäßig gelöst oder dispergiert werden können.

18. Pharmazeutische Arzneiform wie in Anspruch 2 definiert, wobei der Wirkstoff, das Geliermittel, das nicht quellbare Polymer und das Konjugationsmittel unter Verwendung eines pharmazeutisch akzeptablen Lösungsmittels oder einer Mischung von Lösungsmitteln feuchtgranuliert werden.

19. Pharmazeutische Arzneiform wie in Anspruch 1 definiert, wobei die Kapsel ein bis sechs der Minitabletten umfasst, wobei jede 25 bis 75 mg des Wirkstoffs enthält.

20. Pharmazeutische Arzneiform wie in Anspruch 1 definiert, wobei eine Linearität zwischen dem Gesamtgewicht der Minitabletten und der Stärke dieser Arzneiform erreicht wird.

21. Pharmazeutische Arzneiform wie in Anspruch 1 definiert, wobei die Dosis geteilt werden kann, indem die Anzahl der Tabletten in jeder Kapsel reduziert wird.

22. Pharmazeutische Arzneiform wie in Anspruch 1 definiert, die eine Formulierung mit verlängerter Freisetzung für die Verabreichung einmal täglich umfasst, die teilweise oder vollständig mit einer Überzugsschicht oder einem Überzugsfilm beschichtete Minitabletten umfasst, welcher nur während der ersten 2 bis 4 Stunden der Wirkstofffreisetzung funktionell ist.

23. Verfahren zur Herstellung eines Arzneimittel freisetzenden Systems für Venlafaxine, das umfasst: a) Herstellen von Kernen, die Venlafaxine HCL enthalten nach Anspruch 5 durch einen Feuchtgranulierungs-, Trocknungs- und Kompressionsvorgang, b) Auftragen einer funktionellen Überzugsschicht auf die Kerne nach Anspruch 9 unter Verwendung eines direkten Kompressionsvorgangs oder Auftragen eines funktionellen Überzugsfilms auf die Kerne unter Verwendung eines Sprayvorgangs, c) Einkapseln der Minitabletten unter Verwendung einer geeigneten Einkapselungsvorrichtung.

## Revendications

1. Forme galénique pharmaceutique comprenant une formule à libération prolongée de la substance médicamenteuse hydrosoluble Venlafaxine HCl, comprenant une gélule contenant un nombre thérapeutiquement efficace de mini-comprimés comprenant un noyau fonctionnel préparé suivant un procédé de granulation par voie humide, séchage et compression, ainsi qu'une couche d'enrobage fonctionnelle ou une pellicule d'enrobage fonctionnelle, conçue de façon à limiter la libération rapide initiale de la substance médicamenteuse contenue dans les noyaux.

2. Forme galénique pharmaceutique selon la revendication 1, dans laquelle les noyaux des mini-comprimés sont composés de 10 à 40 % en masse de Venlafaxine HCl, de 40 à 80 % en masse d'un gélifiant, de 30 à 60 % en masse d'un agent stabilisant, de 2 à 12 % en masse d'un agent de liaison et de 1 à 30 % en masse d'excipients classiques, à l'exception des excipients qui présentent des propriétés délitantes.

3. Forme galénique pharmaceutique selon la revendication 2, où le gélifiant est un polymère sélectionné au sein du groupe constitué de l'hydroxypropylméthylcellulose, de l'hydroxypropylcellulose, du phtalate d'hydroxycellulose, de l'oxyde de polyéthylène, de l'acide polylactique, de la gomme de xanthane, des alginates, de la carboxyméthylcellulose de sodium et de calcium, du carraghénane, du carbomer, du carbopol pour administration orale, de la méthylhydroxyéthylcellulose, de la propylhydroxyéthylcellulose, des polyHEMA, de la méthylcellulose et des alginates.

4. Forme galénique pharmaceutique selon la revendication 2, où le polymère stabilisant est sélectionné au sein du groupe comprenant l'éthylcellulose, l'acétate propionate de cellulose, l'acétate de cellulose, le poly(acrylate d'éthyle, méthacrylate de méthyle, chlorure de triméthylammonioéthylméthacrylate) 1:2:0,1, commercialisé sous la marque Eudragit RS 100®, le copolymère de poly(acrylate d'éthyle, méthacrylate de méthyle, chlorure de triméthylammonioéthylméthacrylate) 1:2:0,2, disponible dans le commerce sous la marque Eudragit RL®, l'acétate de polyvinylpyrrolidone, le polychlorure de vinyle, le polyacétate de vinyle et le polyéthylène.

5. Forme galénique pharmaceutique selon la revendication 2, où les polymères du noyau sont liés à l'aide d'un agent de liaison de qualité pharmaceutique qui diminue la propension au gonflement du noyau, tel que le laurylsulfate de sodium, le docusate de sodium, le cétostéarylsulfate de sodium et le laurylsulfate de triéthanolamine.

6. Forme galénique pharmaceutique selon la revendication 1, où les noyaux sont en partie enrobés d'une couche d'enrobage fonctionnelle, recouvrant une ou deux surfaces du noyau, ou une surface et le périmètre du noyau, l'épaisseur de la couche d'enrobage variant entre 3 et 30 % du diamètre du noyau.

7. Forme galénique pharmaceutique selon la revendication 6, où la couche d'enrobage fonctionnelle est constituée d'un polymère et d'un composé hydrosoluble, ledit polymère et ledit composé hydrosoluble étant inclus dans un rapport massique compris entre 1:1 et 9:1.

8. Forme galénique pharmaceutique selon la revendication 6, où le polymère est sélectionné soit au sein du groupe constitué de polymères sujets au gonflement tel que ceux cités dans la revendication 3 ci-dessus, soit au sein du groupe constitué de polymères stabilisants, tels que ceux cités dans la revendication 4 ci-dessus.

9. Forme galénique pharmaceutique selon la revendication 6, où le composé hydrosoluble est sélectionné soit au sein du groupe des sels hydrosolubles, tels que le chlorure de sodium et le bicarbonate de sodium, soit au sein du groupe des excipients solides organiques de masse moléculaire relative faible, tels que le mannitol, le lactose, le sucrose, le sorbitol ou l'acide citrique, soit au sein du groupe des polymères hydrosolubles tels que la polyvinylpyrrolidone, l'alcool polyvinylique ou l'hydroxypropylméthylcellulose de faible viscosité.

10. Forme galénique pharmaceutique selon la revendication 1, où les noyaux sont enrobés d'une pellicule d'enrobage fonctionnelle, représentant entre 1,5 et 18 % en masse de la masse du noyau, et appliquée sur une épaisseur suffisante pour limiter la libération initiale de la substance médicamenteuse de la formule.

11. Forme galénique pharmaceutique selon la revendication 10, où la pellicule d'enrobage fonctionnelle comprend un polymère dans une proportion comprise entre 10 et 80 % du matériau d'enrobage sec et un composé hydrosoluble, dans une proportion comprise entre 20 et 50 % du matériau d'enrobage sec.

12. Forme galénique pharmaceutique selon la revendication 11, où le polymère est sélectionné soit au sein du groupe constitué de polymères sujets au gonflement tels que ceux qui sont cités dans la revendication 3, soit au sein du groupe constitué de polymères stabilisants tels que ceux qui sont cités dans la revendication 4, soit au sein du groupe des polymères sensibles au pH qui sont insolubles en milieu acide alors qu'ils ramollissent et se dissolvent en milieu neutre ou basique, tels que l'acétate phtalate de cellulose, le copolymère de poly(méthacrylate de butyle, méthacrylate de (2-diméthylaminoéthyle), méthacrylate de méthyle) 1:2:1, disponible dans le commerce sous la marque Eudragit E®, le copolymère de poly(acrylate d'éthyle, méthacrylate de méthyle) 2:1, disponible dans le commerce sous la marque Eudragit 30D®, le copolymère de poly(acide méthacrylique, méthacrylate de méthyle) 1 :1, disponible dans le commerce sous la marque Eudragit L®, le copolymère de poly(acide méthacrylique, méthacrylate de méthyle) 1:2., disponible dans le commerce sous la marque Eudragit S®.

13. Forme galénique pharmaceutique selon la revendication 11, où le composé hydrosoluble est sélectionné au sein des groupes énumérés dans la revendication 9 ci-dessus.

14. Forme galénique pharmaceutique selon la revendication 1, où la couche ou la pellicule d'enrobage comprend également un agent plastifiant de qualité pharmaceutique.

15. Forme galénique pharmaceutique selon la revendication 1, où la couche d'enrobage comprend en outre des excipients classiques sélectionnés au sein du groupe des agents liants, des agents diluants, des agents glissants, des lubrifiants, des agents d'adhésion, des opacifiants et des colorants.

16. Forme galénique pharmaceutique selon la revendication 1, où la pellicule d'enrobage comprend en outre des excipients classiques sélectionnés au sein du groupe des colorants.

17. Forme galénique pharmaceutique selon la revendication 1, où la pellicule d'enrobage est appliquée à partir d'une solution ou d'une suspension dudit polymère et dudit composé hydrosoluble dans un solvant de qualité pharmaceutique ou un mélange de solvants de qualité pharmaceutique, les constituants de la pellicule d'enrobage sélectionnés pouvant être dissous ou dispersés de façon homogène.

18. Forme galénique pharmaceutique selon la revendication 2, où la substance médicamenteuse, le gélifiant, le polymère stabilisant et l'agent de liaison sont granulés par voie humide en employant un solvant ou un mélange de solvants de qualité pharmaceutique.

19. Forme galénique pharmaceutique selon la revendication 1, où la gélule comprend entre un et six desdits mini-comprimés, chacun contenant entre 25 et 75 mg de la substance médicamenteuse.

20. Forme galénique pharmaceutique selon la revendication 1, où une relation linéaire entre la masse totale des mini-comprimés et l'intensité de l'effet thérapeutique de la Forme galénique pharmaceutique est obtenue.

21. Forme galénique pharmaceutique selon la revendication 1, où la dose peut être divisée en réduisant le nombre de comprimés dans chaque gélule.

22. Forme galénique pharmaceutique selon la revendication 1, comprenant une formule à libération prolongée pour une administration journalière à raison d'une fois par jour, ladite formule comprenant des mini-comprimés partiellement ou totalement enrobés d'une couche d'enrobage ou d'une pellicule d'enrobage qui n'est fonctionnelle que durant les premières 2 à 4 heures de la libération du médicament.

23. Méthode d'élaboration d'un système de libération de médicament pour la Venlafaxine, qui comprend les étapes suivantes : a) préparation des noyaux contenant la Venlafaxine HCl selon la revendication 5 par un procédé de granulation par voie humide, séchage et compression, b) application d'une couche d'enrobage fonctionnelle sur les noyaux selon la revendication 9, en employant un procédé de compression directe, ou application d'une pellicule d'enrobage fonctionnelle sur les noyaux en employant un procédé de vaporisation, c) encapsulation des mini-comprimés en employant un dispositif d'encapsulation approprié.
